# EUROPEAN PATENT SPECIFICATION

(11) **EP 0 746 338 B1**
(45) Date of publication and mention of the grant of the patent: **27.10.2004**
(21) Application number: 95907423.8
(22) Date of filing: 11.01.1995
(51) Int. Cl.: A61K 39/145, A61K 39/39, A61K 9/127

(54) **VACCINES CONTAINING PAUCILAMELLAR LIPID VESICLES AS IMMUNOLOGICAL ADJUVANTS**
Impfstoffe, die paucilamelläre Lipidvesikel als Adjuvans enthalten
VACCINS CONTENANT DES VESICULES LIPIDIQUES PAUCILAMELLAIRES COMME ADJUVANTS IMMUNOLOGIQUES

(30) Priority: 24.02.1994 US 201346
(43) Date of publication of application: 11.12.1996
(73) Proprietor: MICRO-PAK, INC., Wilmington, DE 19810 (US)
(72) Inventor: WRIGHT, Craig D., Gaithersburg, MD 20878 (US); WALLACH, Donald F.H., Hollis, NH 03049 (US)
(74) Representative: Kirkham, Nicholas Andrew
(86) International application number: PCT/US1995/000475
(87) International publication number: WO 1995/022989

(56) References cited:
- EP-A- 0 356 339
- EP-A- 0 356 340
- WO-A-94/16061
- US-A- 4 241 046
- US-A- 4 826 687
- US-A- 4 911 928
- US-A- 5 000 960
- US-A- 5 032 457
- US-A- 5 100 662
- US-A- 5 147 723
- US-A- 5 234 767

## Description

### Background of the Invention

The present invention relates to an adjuvanted vaccine, where lipid vesicles, particularly nonphospholipid lipid vesicles, serve as the adjuvant, together with methods of preparing the vaccine. Immunological adjuvants are the component of the vaccine which augment the immune response to the antigen. Immunological adjuvants function by, inter alia, attracting macrophages to the antigen and then to present that antigen to the regional lymph nodes and initiate an effective antigenic response. Adjuvants may also act as carriers themselves for the antigen. Many of the known immunological adjuvants, such as Freund's complete adjuvant, alum, aluminum hydroxides, and Freund's incomplete adjuvant, while effective at initiating the antigenic response, produce undesirable reactions in humans, such as inflammation at the point of injection. These side effects prevent use of such adjuvants in humans, and have led to the search for alternative immunological adjuvants.

Lipid vesicles are substantially spherical structures made of amphiphiles, e.g., surfactants or phospholipids. The lipids of these spherical vesicles are generally organized in the form of lipid bilayers, e.g., multiple onion-like shells of lipid bilayers which encompass an aqueous volume between the bilayers. Certain types of lipid vesicles have an unstructured central cavity which can be used to encapsulate and transport a variety of materials. Paucilamellar lipid vesicles, for example, have 2-10 peripheral bilayers surrounding a large, unstructured central cavity.

Until recently, liposome technology has been concerned mostly with vesicles composed of phospholipids. This is primarily because phospholipids are the principal structural components of natural membranes and, accordingly, lipid vesicles have been used as a model system for studying natural membranes. However, there are a number of problems associated with using phospholipids as synthetic membranes. Phospholipid liposomes placed in an in vivo environment are rapidly degraded. Moreover, phospholipids are labile and expensive to purify or synthesize. In addition, classic phospholipid liposomes are in the form of multilamellar as opposed to paucilamellar vesicles and have poor carrying capacities, especially for lipophilic materials, and have poor shelf lives unless lyophilized in the dark with antioxidants. Finally, phospholipids degrade too rapidly in vivo for most pharmaceutical or vaccine applications.

For these reasons, there is increasing interest in liposomes made of commercially available nonphospholipid amphiphiles (see, e.g., U.S. Pat. No. 4,217,344, U.S Pat. No. 4,917,951, and U.S. Pat. No. 4,911,928). These molecules have a hydrophilic head group attached to a hydrophobic "tail" and are derived from long chain fatty acids, long chain alcohols and their derivatives, long chain amines, and polyol sphingo- and glycerolipids. Commercially available amphiphile surfactants include, for example, the BRIJ family of polyoxyethylene fatty ethers, the SPAN sorbitan fatty acid esters, and the TWEEN polyoxyethylene derivatives of sorbitan fatty acid esters; all available from ICI Americas, Inc. of Wilmington, De. Paucilamellar vesicles containing such amphiphiles provide a high carrying capacity for water-soluble and water immiscible substances. The high capacity for water immiscible substances represents a unique advantage over classical phospholipid multilamellar liposomes.

Paucilamellar lipid vesicles may include a wide variety of phospholipids and nonphospholipid surfactants as their primary structural material. Paucilamellar lipid vesicles are substantially spherical structures made of materials having a high lipid content, preferably from nonphospholipid materials, which are organized in the form of lipid bilayers. The two to ten peripheral bilayers encapsulate an aqueous volume which is interspersed between the lipid bilayers and may also be encapsulated in the amorphous central cavity. Alternatively, the amorphous central cavity may be substantially filled with a water immiscible material, such as an oil or wax. Paucilamellar lipid vesicles have advantages as transport vehicles because large unstructured central cavity is easily adaptable for transport of large quantities of aqueous or oleaginous materials.

As described above, to stimulate a specific immune response, two components are required, namely the antigen or immunologicaly specific substance, and an adjuvant, the component augmenting the immune response to the antigen. Conventional adjuvants can serve as vehicles for the antigen, and as nonspecific immunological stimulants. The inventors have discovered that paucilamellar lipid vesicles are effective immunological adjuvants.

Accordingly, it is an object of the invention to provide an adjuvanted vaccine for immunizing against influenza, where paucilamellar lipid vesicles are the adjuvant.

Another object of the invention is to provide an adjuvanted vaccine to stimulate an immune response in a mammal, where the adjuvant is a nonphospholipid paucilamellar lipid vesicle which acts as a non-specific immune stimulator, an adjuvant/antigen carrier or as a carrier of chemical adjuvants.

A further object of the invention is to provide a method of preparing adjuvanted vaccines useful in treating viral infections in mammals.

These and other objects and features of the invention will be apparent from the following description and from the claims.

### Summary of the Invention

In a first aspect the invention relates to a vaccine for producing an antigenic response to influenza *in vivo,* in mammals, said vaccine comprising:
an antigenically effective amount of an influenza virus antigen derived from formalin-inactivated whole virus or subunits thereof and an adjuvant, said adjuvant comprising paucilamellar lipid vesicles having nonphospholipid materials as the primary wall forming constituent, wherein said paucilamellar lipid vesicles have 2-10 bilayers surrounding an amorphous cental cavity, and
wherein said nonphospholipid materials are selected from the group consisting of polyoxyethylene fatty acid esters, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures of said non-phospholipid materials.

In a further aspect the invention relates to the use for the manufacture of a vaccine for immunizing a mammal against influenza, of an influenza virus antigen derived from formalin inactivated whole virus or subunits thereof and an adjuvant, wherein said adjuvant comprises nonphospholipid paucilamellar lipid vesicles, said nonphospholipid paucilamellar lipid vesicles have 2-10 bilayers surrounding an amorphous central cavity and comprising nonphospholipid materials selected from the group consisting of polyoxyethylene fatty acid esters, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures of said non-phospholipid materials, said vaccine being in a form suiting administration intramuscularly, intraperitonealy or orally.

In another aspect the invention relates to a method of preparing an adjuvanted influenza vaccine said method comprising the steps of:
forming an adjuvant comprising nonphospholipid paucilamellar lipid vesicles having 2-10 lipid bilayers surrounding an amorphous central cavity, said nonphospholipid paucilamellar lipid vesicles comprising nonphospholipid materials selected from the group consisting of polyoxyethylene fatty acid ester, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures of said non-phospholipid materials; and mixing said adjuvant with an influenza virus antigen derived from formalin inactivated whole virus or subunits thereof.

In a further aspect the invention relates to a method of preparing an adjuvanted influenza vaccine said method comprising the steps of:
preparing a lipophilic phase containing a nonphospholipid material wherein said nonphospholipid material is selected from the group consisting of polyoxyethylene fatty acid esters, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures of said non-phospholipid materials;
preparing an aqueous phase containing an influenza virus antigen derived from formalin inactivated whole virus or subunits thereof; and
shear mixing said lipophilic phase with said aqueous phase to form paucilamellar lipid vesicles having 2-10 lipid bilayers surrounding an amorphous central cavity, wherein said antigen is encapsulated in said cavity.

In a further preferred aspect the invention relates to a method of forming an adjuvanted vaccine wherein said adjuvant comprises a paucilamellar lipid vesicle having a substantially amorphous central cavity containing a water-immiscible oily material, 2-10 bilayers surrounding said amorphous central cavity and an influenza virus antigen, said paucilamellar lipid vesicle comprising nonphospholipid materials selected from the group consisting of polyoxyethylene fatty acid esters, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures thereof, said method comprising the steps of:
preforming a paucilamellar lipid vesicle having an aqueous material in the amorphous central activity, said paucilamellar lipid vesicle comprises nonphospholipid materials selected from the group consisting of polyoxyethylene fatty acid esters, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures of said non-phospholipid materials; and
mixing under shear mixing conditions, said preformed paucilamellar lipid vesicle with a water-immiscible oily material containing an influenza virus antigen to be incorporated into said central cavity such that said water immiscible material and said influenza virus antigen are incorporated into said preformed vesicle, whereby said amorphous central cavity of said paucilamellar lipid vesicle is substantially filled with said water immiscible material.

The present invention features an adjuvanted vaccine and methods for preparing an adjuvanted vaccine, preferably for immunizing against influenza, where the adjuvant is a lipid vesicle and preferably is a nonphospholipid, paucilamellar lipid vesicle. The antigen may be encapsulated in the central cavity of the adjuvant or mixed in solution with the adjuvant. Moreover, the adjuvant may carry a secondary adjuvant to further improve the immune response.

The antigen is preferably an influenza antigen and may comprise a formalin-inactivated whole virus, formalin-inactivated viral subunits or an antigen produced by recombinant DNA techniques.

In one embodiment, the adjuvanted flu vaccine is prepared whereby the paucilamellar lipid vesicles, the preferred adjuvant, are prepared separately and the adjuvant is then intermixed with the antigen. Alternatively, an adjuvanted vaccine can be prepared by forming paucilamellar lipid vesicles encapsulating the antigen.

The adjuvant in one embodiment of the invention is a paucilamellar lipid vesicle having about two to ten bilayers arranged in the form of substantially spherical shells separated by aqueous layers surrounding a large amorphous central cavity free of lipid bilayers. The lipid bilayers preferably have as their primary lipid component one or more of the following nonphospholipid materials: polyoxyethylene fatty acid esters, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures thereof. More preferably, this mixture further contains at least one sterol selected from the group consisting of cholesterol, cholesterol derivatives, hydrocortisone, phytosterol, and mixtures thereof, a charge producing agent, and any lipid soluble or water soluble materials to be incorporated into the vesicles.

The vesicles of the present invention have a central cavity, carrying either water soluble materials or a water-immiscible oily solution, which can be used to encapsulate the antigen. The water-immiscible oily solution is made of materials which are both water immiscible and immiscible in the lipids used to form the bilayers. The water immiscible oily material found the amorphous central cavity may comprise soybean oil, squalene oil, squalane oil, sesame oil, olive oil, canola oil, corn oil, rapeseed oil, safflower oil, sunflower oil, fish oils, petrolatum, avocado oil, triglyceride oils and fats, flavor oils, water insoluble vitamins, and mixtures thereof. These materials provide pharmacological benefits in addition to the benefits caused by the use of the particular lipids which form the bilayers.

The invention further features methods of producing adjuvanted vaccines. The adjuvant may comprise water or oil filled vesicles, e.g., vesicles having their amorphous central cavities filled with a water-immiscible oily solution, and these may be formed using either the "hot loading" technique disclosed in Unites States Patent No. 4,911,928 or the "cold loading" technique described in the United States Patent No. 5,160,669. In either case, a lipid phase is formed by blending the nonphospholipid material, along with any sterols or lipophilic materials to be incorporated into the lipid bilayers, to form a homogenous lipid phase. In the "hot loading" technique, any water-immiscible oily material to be encapsulated in the vesicles is blended in the already formed lipid phase, forming a lipophilic phase. Oil-soluble or oil-suspendable antigens to be encapsulated within the vesicles are first dispersed in the oil. The term "dispersed" as used herein includes dissolution or forming a suspension or colloid to yield a flowable phase.

Once a lipophilic phase is made, it is blended with an aqueous phase (e.g., water, saline, or any other aqueous solution which will be used to hydrate the lipids), which may also contain an antigen, under shear mixing conditions to form the adjuvant. "Shear mixing conditions", as used herein, means a shear equivalent to a relative flow of 5-50 m/s through a 1mm orifice.

In the alternative, the vaccine can be incorporated into the amorphous central cavity of the adjuvant by the "cold-loading" technique described in U.S. Patent No. 5,160,669 to Wallach et al.

The scope and application of the invention will be apparent from the following detailed description.

### Brief Description of the Drawings

FIG. 1 is a graph of the mean IFA results in mice at day 42 following one inoculation with adjuvanted influenza A vaccines;
FIG. 2 illustrates the mean IFA values in rabbits at day 27 following two inoculations with adjuvanted influenza A vaccines;
FIG. 3 illustrates the mean HI values in rabbits at day 27 following two inoculations with adjuvanted influenza A vaccines.

### Detailed Description of the Invention

The present invention involves use of paucilamellar lipid vesicles as adjuvants in a vaccine to increase the antigenic response in a mammal inoculated with the vaccine. The vesicles are preferably nonphospholipid vesicles, and the antigen is preferably an influenza antigen.

Paucilamellar lipid vesicles act to stimulate the immune response several ways, as non-specific stimulators, as carriers for the antigen, as carriers of additional adjuvants, and combinations thereof. Paucilamellar lipid vesicles act as non-specific immune stimulators when, for example, a vaccine is prepared by intermixing the antigen with the preformed vesicles such that the antigen remains extracellular to the vesicles. By encapsulating an antigen within the central cavity of the vesicle, the vesicle acts both as an immune stimulator and a carrier for the antigen. Alternatively, the vesicles can act as carriers for the antigen by fusing with the antigen, as is described in WO 9416061 , entitled *Method of Inhibiting Viral Reproduction*. In this embodiment, when the antigen, there an enveloped virus, is mixed with the paucilamellar lipid vesicles, the virus and adjuvant fuse, denaturing the nucleic acid and inactivating the virus. The inactivated virus/adjuvant hybrid is then useful as a vaccine. Moreover, the vesicle can serve to carry additional adjuvants within the central cavity or between the bilayers.

The following Examples will clearly illustrate the efficacy of the invention.

### Example 1:

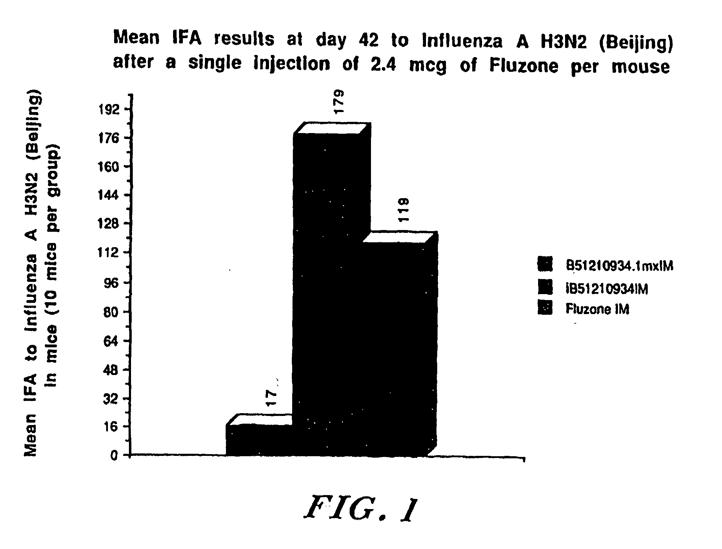

An adjuvanted vaccine containing the antigen influenza A H3N2 (Beijing) was prepared using nonphospholipid paucilamellar lipid vesicles as adjuvants. Adjuvanticity of the two formulations, namely, non-specific immune stimulator and carrier adjuvant formulations was compared using the mean Indirect Fluorescent Assay (IFA) of each composition, as compared with that of the antigen alone, as shown in Figure 1.

Adjuvant formulations were prepared using an automated syringe machine, specifically a 5cc syringe machine. The adjuvant could also be made according to the general procedure set forth in United States Patent No. 4,911,928. Briefly, the lipid components of the vesicle walls were heated to a flowable state and placed in a first component of the syringe machine. The aqueous component, in this case containing the antigen Fluzone™ (see below), was heated and placed in a second component of the syringe machine. The materials were then mixed using shear mixing until vesicles formed, encapsulating the antigen in the central cavity. However, in this and the following Examples, any method of achieving the proper shear could be used, including the manual techniques described in U.S. Patent No. 4,911,928 (two syringes connected via a stopcock), or a flow device such as the NovaMix™ vesicle former. The basic details of the NovaMix™ system are described in United States Patent No. 4,895,452.

The antigen used in this example was Fluzone™ a formalin-inactivated detergent-extracted influenza vaccine from Connaught. The formulation for the adjuvants used in this Example are set forth in Tables 1 and 2 below.

**TABLE 1**

| | |
|---|---|
| Lipid Formulation | Brij 52 (7.0g); cholesterol (2.69g) |
| Diluent | Water for injection (WFI) containing 2.4 µg/ml Fluzone |
| Diluent Volume | 4.0ml |
| Charge | Negative (Oleic acid 0.31g) |
| Oil | Squalene |
| Hydration Ratio | 1.6/1 (lipid/sqe) |
| | 1.0/4.0 (lipid, oil/Diluent) |
| Temperature of WFI Phase | 60° C |
| Temperature of Lipid Phase | 85° C |
| pH | 5.85 |
| Final Volume | 5 ml |

For the first vaccine preparation, where the adjuvant encapsulates the antigen, the vaccine was made according to the formula of Table 1. The second vaccine preparation is made according to the formula of Table 2 below, where the diluent is water, without the antigen.

**TABLE 2**

| | |
|---|---|
| Lipid Formulation | Brij 52 (7.0g); cholesterol (2.69g) |
| WFI | Water for injection (WFI) |
| Diluent Volume | 4.0ml |
| Charge | Negative (Oleic acid 0.31g) |
| Oil | Squalene |
| Hydration Ratio | 1.6/1 (lipid/sqe); |
| | 1.0/4.0 (lipid, oil/Diluent) |
| Temperature of WFI Phase | 60° C |
| Temperature of Lipid Phase | 85° C |
| pH | 5.85 |
| Final Volume | 5 ml |

The adjuvant for the second vaccine preparation is prepared according to the method described above and then diluted 1:10. Of that diluted adjuvant, 100 µl are added to 2.4 µg of the Fluzone antigen for injection into each animal.

Three groups of ten C₃H seven week old female mice where injected with each vaccine preparation, resulting in 2.4 micrograms of antigen given per mouse. The first group of mice received one injection of the antigen alone; the second group received one injection of the antigen incorporated into the adjuvant; and the third group of mice received one injection of the antigen intermixed with the one to ten dilution of adjuvant. As can be seen from FIG. 1, which illustrates mean IFA results at day 42, the adjuvanted vaccines improved the antigenic response significantly over the antigen alone. The adjuvant encapsulating the antigen exhibits a log increase over the antigen alone, and the diluted adjuvant exhibits a 7/10 log increase.

### Example 2:

In this example, New Zealand Albino rabbits from Hazelton Labs were immunized with adjuvanted influenza A (Beijing) H3N2 vaccines to compare the adjuvant formulations of the present invention with the antigen alone, and with two other adjuvants not suitable for use in humans.

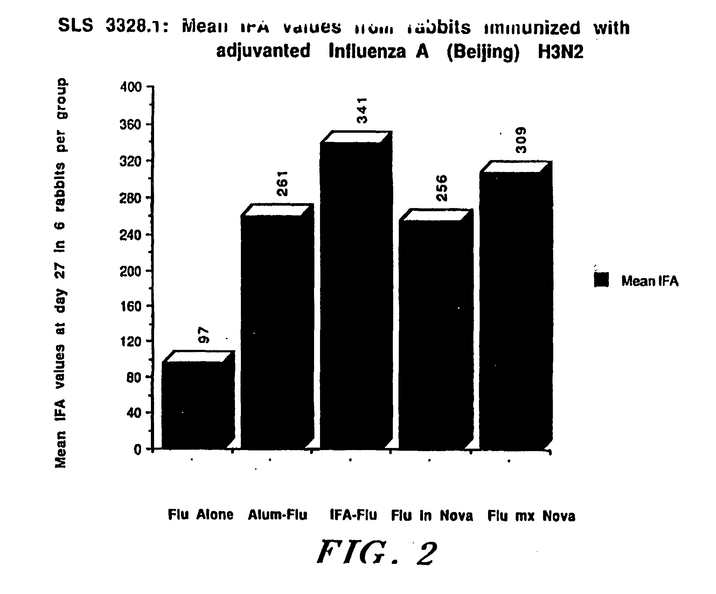

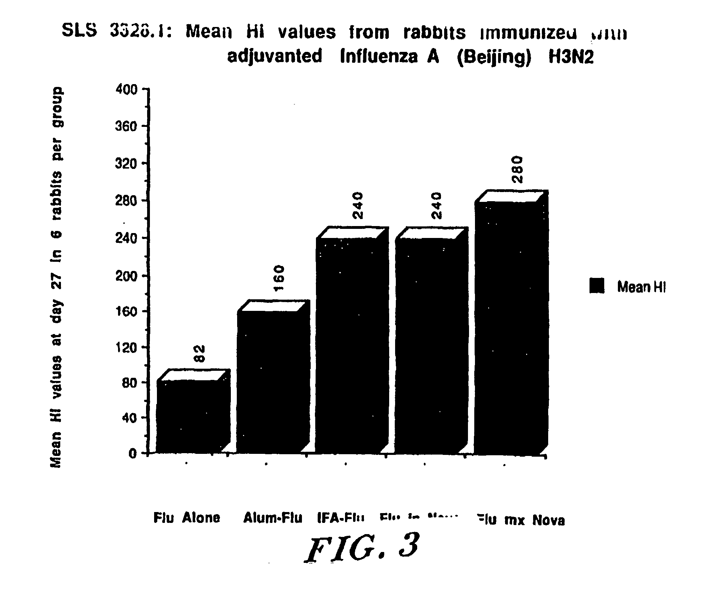

Each group of six rabbits (three males and three females) was injected with 9.8 micrograms of influenza A H3N2 antigen per animal. This antigen is a whole virus preparation produced in chicken eggs, which has been formalin-inactivated and purified by centrifugation and column filtration. In each case, one half milliliter of the vaccine was injected intramuscularly into each rabbit at days 0 and 14. The data from Figures 2 and 3 was determined from a bleeding taken on day 27.

The first group of rabbits received the antigen alone, the second group of rabbits received the antigen adjuvanted with 16 µg alum/1µg protein (Resorptar Armour Pharmaceuticals). The third group received the antigen adjuvanted with a 1:1 mixture (vol/vol) incomplete Freund's (Sigma Chemical).

The fourth group of rabbits received the antigen encapsulated in paucilamellar lipid vesicles prepared according to the formula set out in Table 3 below, prepared as described in Example 1, and the fifth group received 9.8 µg antigen in solution 1:1 (vol/vol) with adjuvant, specifically the paucilamellar lipid vesicles prepared according to the formulation set forth in Table 4 below.

**TABLE 3**

| | |
|---|---|
| Lipid Formulation | Brij 52 (17.5g), cholesterol (6.4g) |
| Diluent | Water for injection (WFI) with 9.8 µg antigen |
| Diluent Volume | 3.7ml |
| Charge | None |
| Oil | Soybean oil |
| Hydration Ratio | (1.4ml lipid/1ml oil); |
| | 1.3/3.7 (lipid/oil to WFI) |
| Temperature of WFI Phase | 56°C |
| Temperature of Lipid Phase | 74° C |
| pH | 6.6 |
| Final Volume | 5 ml |

**TABLE 4**

| | |
|---|---|
| Lipid Formulation | Brij 52 (17.5g), cholesterol (6.4g) |
| Diluent | Water for injection (WFI) |
| Diluent Volume | 3.7ml |
| Charge | None |
| Oil | Soybean oil |
| Hydration Ratio | (1.4ml lipid/1ml oil); |
| | 1.3/3.7 (lipid/oil to WFI) |
| Temperature of WFI Phase | 56° C |
| Temperature of Lipid Phase | 74° C |
| pH | 6.6 |
| Final Volume | 5 ml |

As can be seen from Figures 2 and 3, the adjuvanted vaccine according the present invention has equivalent or increased antibody response and antigenicity when compared with that of known adjuvants. The mean IFA results were calculated as described above. The mean HI (Hemagglutination Inhibition) assay values were obtained from testing with chicken red blood cells, as is known in the art, the results of which correlate with protection capabilities of the vaccine.

The foregoing Examples are merely illustrative and those skilled in the art may be able to determine other materials and methods which accomplish the same result. Such other materials and methods are included within the scope of the following claims.What is claimed is:

## Claims

1. A vaccine for producing an antigenic response to influenza, *in vivo,* in mammals, said vaccine comprising:
an antigenically effective amount of an influenza virus antigen derived from formalin-inactivated whole virus or subunits thereof and an adjuvant, said adjuvant comprising paucilamellar lipid vesicles having nonphospholipid materials as the primary wall forming constituent, wherein said paucilamellar lipid vesicles have 2-10 bilayers surrounding an amorphous central cavity, and
wherein said nonphospholipid materials are selected from the group consisting of polyoxyethylene fatty acid esters, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures of said non-phospholipid materials.

2. The vaccine of claim 1 wherein said antigen and said adjuvant are intermixed in said vaccine, or said antigen is encapsulated in said adjuvant, e.g. is encapsulated in said amorphous central cavity.

3. The vaccine of claim 1 wherein said antigen is selected from the group consisting of antigens derived from formalin-inactivated whole virus, antigens derived from formalin-inactivated viral subunits and e.g.
said antigen is selected from the group consisting of formalin inactivated detergent extracted influenza viruses and influenza A H3N2.

4. The vaccine of claim 1 wherein said paucilamellar lipid vesicles further comprise at least one sterol selected from the group consisting of cholesterol, cholesterol derivatives, hydrocortisone, phytosterol and mixtures thereof.

5. The vaccine of claim 1 wherein said paucilamellar lipid vesicles comprise an amorphous central cavity containing a water immiscible oily material, e.g.
selected from the group consisting of soybean oil, sqaulene oil, sesame oil, olive oil, canola oil, corn oil, rapeseed oil, safflower oil, sunflower oil, fish oils, petrolatum, avocado oil, triglyceride oils and fats, flavor oils, water insoluble vitamins and mixtures thereof.

6. Use, for the manufacture of a vaccine for immunizing a mammal against influenza, of an influenza virus antigen derived from formalin inactivated whole virus or subunits thereof and an adjuvant, wherein said adjuvant comprises nonphospholipid paucilamellar lipid vesicles, said nonphospholipid paucilamellar lipid vesicles have 2-10 bilayers surrounding an amorphous central cavity and comprising nonphospholipid materials selected from the group consisting of polyoxyethylene fatty acid esters, polyoxyethylene fatty acid ethers, polyoxyethylene sorbitan esters, polyoxyethytene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures of said non-phospholipid materials, said vaccine being in a form suiting administration intramuscularly, intraperitonealy or orally.

7. The use of claim 6 wherein said antigen and said adjuvant are intermixed in said vaccine, or said antigen is encapsulated in said adjuvant.

8. The use of claim 6 wherein said antigen is encapsulated in said amorphous central cavity.

9. The use of claim 6 wherein said antigen is selected from the group consisting of antigens derived from formalin-inactivated whole virus, antigens derived from formalin-inactivated viral subunits and e.g.
said antigen is selected from the group consisting of formalin inactivated detergent extracted influenza viruses and influenza A H3N2.

10. The use of claim 6 wherein said paucilamellar lipid vesicles further comprise at least one sterol selected from the group consisting of cholesterol, cholesterol derivatives, hydrocortisone, phytosterol and mixtures thereof.

11. The use of claim 6 wherein said paucilamellar lipid vesicles comprise an amorphous central cavity containing a water immiscible oily material, e.g. selected from the group consisting of soybean oil, squalene oil, sesame oil, olive oil, canola oil, corn oil, rapeseed oil, safflower oil, sunflower oil, fish oils, petrolatum, avocado oil, triglyceride oils and fats, flavor oils, water insoluble vitamins and mixtures thereof.

12. A method of preparing an adjuvanted influenza vaccine, said method comprising the steps of:
forming an adjuvant comprising nonphospholipid paucilamellar lipid vesicles having 2-10 lipid bilayers surrounding an amorphous central cavity, said nonphospholipid paucilamellar lipid vesicles comprising nonphospholipid materials selected from the group consisting of polyoxyethylene fatty acid ester, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures of said non-phospholipid materials; and mixing said adjuvant with an influenza virus antigen derived from formalin inactivated whole virus or subunits thereof.

13. The method of claim 12 wherein said antigen is encapsulated in said amorphous central cavity of said adjuvant.

14. A method of preparing an adjuvanted influenza vaccine, said method comprising the steps of:
preparing a lipophilic phase containing a nonphospholipid material wherein said nonphospholipid material is selected from the group consisting of polyoxyethylene fatty acid esters, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures of said non-phospholipid materials;
preparing an aqueous phase containing an influenza virus antigen derived from formalin inactivated whole virus or subunits thereof; and
shear mixing said lipophilic phase with said aqueous phase to form paucilamellar lipid vesicles having 2-10 lipid bilayers surrounding an amorphous central cavity, wherein said antigen is encapsulated in said cavity.

15. The method of claim 12 or claim 14, wherein said amorphous central cavity of said paucilamellar lipid vesicles contains a water immiscible oily material, e.g. selected from the group consisting of soybean oil, squalene oil, sesame oil, olive oil, canola oil, corn oil, rapeseed oil, safflower oil, sunflower oil, fish oils, petrolatum, avocado oil, triglyceride oils and fats, flavor oils, water insoluble vitamins and mixtures thereof.

16. A method of forming an adjuvanted vaccine wherein said adjuvant comprises a paucilamellar lipid vesicle having a substantially amorphous central cavity containing a water-immiscible oily material, 2-10 bilayers surrounding said amorphous central cavity and an influenza virus antigen derived from formalin inactivated whole virus or subunits thereof said paucilamellar lipid vesicle comprising nonphospholipid materials selected from the group consisting of polyoxyethylene fatty acid esters, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures thereof, said method comprising the steps of:
preforming a paucilamellar lipid vesicle having an aqueous material in the amorphous central cavity said paucilamellar lipid vesicle comprises nonphospholipid materials selected from the group consisting of polyoxyethylene fatty acid esters, polyoxyethylene fatty acid ethers, polyoxyethylene sorbitan esters, polyoxyethylene glyceryl mono- and diesters, glyceryl mono- and distearate, sucrose distearate, propylene glycol stearate, long chain acyl hexosamides, long chain acyl amino acid amides, long chain acyl amides, glyceryl mono- and diesters, dimethyl acyl amines, C₁₂-C₂₀ fatty alcohols, C₁₂-C₂₀ glycol monoesters, C₁₂-C₂₀ fatty acids and mixtures of said non-phospholipid materials; and
mixing under shear mixing conditions, said preformed paucilamellar lipid vesicle with a water-immiscible oily material containing an influenza virus antigen to be incorporated into said central cavity such that said water immiscible material and said influenza virus antigen are incorporated into said preformed vesicle, whereby said amorphous central cavity of said paucilamellar lipid vesicle is substantially filled with said water immiscible material.

17. The method of claim 16 further comprising the step of separating said paucilamellar lipid vesicle from any of said water immiscible material and antigen not incorporated into said paucilamellar lipid vesicle.

18. The method of claim 16 wherein said paucilamellar lipid vesicles have 2-10 bilayers surrounding an amorphous central cavity.

19. The method of claim 12, 14 or 16, wherein said antigen is selected from the group consisting of antigens derived from formalin-inactivated whole virus, antigens derived from formalin-inactivated viral subunits and e.g. said antigen is selected from the group consisting of formalin inactivated detergent extracted influenza viruses and influenza A H3N2.

20. The method of claim 12,14 or 16 wherein said paucilamellar lipid vesicle or said lipophilic phase further comprise at least one sterol selected from the group consisting of cholesterol, cholesterol derivatives, hydrocortisone, phytosterol and mixtures thereof.

21. The method of claim 16 wherein said water immiscible oil material is selected from the group consisting of soybean oil, squalene oil, sesame oil, olive oil, canola oil, corn oil, rapeseed oil, safflower oil, sunflower oil, fish oils, petrolatum, avocado oil, triglyceride oils and fats, flavor oils, water insoluble vitamins and mixtures thereof.

## Patentansprüche

1. Impfstoff zur Erzeugung einer antigenen Reaktion auf Influenza in vivo in Säugern, wobei der Impfstoff aufweist:
eine antigen wirksame Menge eines Influenzavirusantigens, abgeleitet aus einem formalindeaktivierten Gesamtvirus, oder Untereinheiten davon, und ein Hilfsstoff, wobei der Hilfsstoff paucilamellare Lipidvesikel mit Nichtphpospholipidmaterialienal als der primäre, wandbildende Bestandteil aufweist, wobei die paucilamellaren Lipidvesikel 2 bis 10 Doppelschichten aufweisen, die einen amorphen, zentralen Hohlraum umschließen, und
wobei die nicht Phospholipidmaterialien ausgewählt sind aus der Gruppe, bestehend aus Polyoxyethylenfettsäurestern, Polyoxyethylenfettsäurethern, Polyoxyethylensorbitanestern, Polyoxyethylengycerylmono- und Diestern, Glycerylmono- und Distearat, Saccharosedistearat, Propylenglycolstearat, langkettige Acylhexosamide, langkettige Acylaminosäureamide, langkettige Acylamide, Glycerylmono- und Diester, Dimethylacylamine, C₁₂ bis C₂₀ Fettalkohole, C₁₂ bis C₂₀ Glycolmonoester, C₁₂ bis C₂₀ Fettsäuren und Mischungen dieser Nichtphospholipidmaterialien.

2. Impfstoff nach Anspruch 1, **dadurch gekennzeichnet, dass** das Antigen und der Hilfsstoff im Impfstoff vermischt werden oder, dass das Antigen in dem Hilfsstoff eingekapselt ist, zum Beispiel in den amorphen, zentralen Hohlraum eingekapselt ist.

3. Impfstoff nach Anspruch 1, **dadurch gekennzeichnet, dass** das Antigen ausgewählt ist aus der Gruppe, bestehend aus Antigenen, die aus formalindeaktivierten Gesamtviren abgeleitet sind, Antigenen, die aus formalindeaktivierten Virusuntereinheiten abgeleitet sind und beispielsweise das Antigen ausgewählt ist aus der Gruppe, bestehend aus formalindeaktivierten, mit Detergenzien extrahierten Influenzaviren und Influenza A H3N2.

4. Impfstoff nach Anspruch 1, **dadurch gekennzeichnet, dass** die paucilamellaren Lipidvesikel des weiteren zumindest ein Sterin, ausgewählt aus der Gruppe, bestehend aus Cholesterin, Cholesterinderivaten, Hydrocortison, Phytosterin und Mischungen daraus, aufweisen.

5. Impfstoff nach Anspruch 1, **dadurch gekennzeichnet, dass** die paucilamellaren Lipidvesikel einen zentralen, amorphen Hohlraum aufweisen, der ein mit Wasser nicht mischbares, öliges Material enthält, beispielsweise ausgewählt aus der Gruppe, bestehend aus Sojabohnenöl, Squalenöl, Sesamöl, Olivenöl, Canolaöl, Maisöl, Rapsöl, Distelöl, Sonnenblumenöl, Fischölen, Petrolat, Avocadoöl, Triglycerid Öle und Fette, Duftöle, wasserunlöslichen Vitaminen und Mischungen daraus.

6. Verwendung eines Influenzavirusantigens, abgeleitet aus formalindeaktivierten Gesamtviren oder Untereinheiten davon und einem Hilfsstoff, zur Herstellung eines Medikaments zur Immunisierung eines Säugers gegen Influenza, wobei der Hilfsstoff nichtphpospholipid paucilamellare Lipidvesikel aufweist, wobei die paucilamellaren Lipidvesikel 2 bis 10 Doppelschichten aufweisen, die einen amorphen, zentralen Hohlraum umschließen, und die Nichtphospholipidmaterialien, ausgewählt aus der Gruppe, bestehend aus Polyoxyethylenfettsäurestern, Polyoxyethylenfettsäurethern, Polyoxyethylensorbitanestern, Polyoxyethylengycerylmono- und Diestern, Glycerylmono- und Distearat, Saccharosedistearat, Propylenglycolstearat, langkettige Acylhexosamide, langkettige Acylaminosäureamide, langkettige Acylamide, Glycerylmono- und Diester, Dimethylacylamine, C₁₂ bis C₂₀ Fettalkohole, C₁₂ bis C₂₀ Glycolmonoester, C₁₂ bis C₂₀ Fettsäuren und Mischungen dieser Nichtphospholipidmaterialien, aufweisen, wobei der Impfstoff sich in einer Form befindet, die sich für eine intramuskuläre, intraperitoneale und orale Verabreichung eignet.

7. Verwendung nach Anspruch 6, **dadurch gekennzeichnet, dass** das Antigen und der Hilfsstoff im Impfstoff vermischt werden oder, dass das Antigen in dem Hilfsstoff eingekapselt ist,

8. Verwendung nach Anspruch 6, **dadurch gekennzeichnet, dass** das Antigen in den amorphen, zentralen Hohlraum eingekapselt ist.

9. Verwendung nach Anspruch 6, **dadurch gekennzeichnet, dass** das Antigen ausgewählt ist aus der Gruppe, bestehend aus Antigenen, die aus formalindeaktivierten Gesamtviren abgeleitet sind, Antigenen, die aus formalindeaktivierten Virusuntereinheiten abgeleitet sind und beispielsweise das Antigen ausgewählt ist aus der Gruppe, bestehend aus formalindeaktivierten, mit Detergenzien extrahierten Influenzaviren und Influenza A H3N2.

10. Verwendung nach Anspruch 6, **dadurch gekennzeichnet, dass** die paucilamellaren Lipidvesikel des weiteren zumindest ein Sterin, ausgewählt aus der Gruppe, bestehend aus Cholesterin, Cholesterinderivaten, Hydrocortison, Phytosterin und Mischungen daraus, aufweisen.

11. Verwendung nach Anspruch 6, **dadurch gekennzeichnet, dass** die paucilamellaren Lipidvesikel einen zentralen, amorphen Hohlraum aufweisen, der ein mit Wasser nicht mischbares, öliges Material enthält, beispielsweise ausgewählt aus der Gruppe, bestehend aus Sojabohnenöl, Squalenöl, Sesamöl, Olivenöl, Canolaöl, Maisöl, Rapsöl, Distelöl, Sonnenblumenöl, Fischölen, Petrolat, Avocadoöl, Triglycerid Öle und Fette, Duftöle, wasserunlöslichen Vitaminen und Mischungen daraus.

12. Verfahren zur Herstellung eines Influenzaimpfstoffs mit Hilfsstoff, wobei man:
einen Hilfsstoff, der nichtphpospholipid paucilamellare Lipidvesikel mit 2 bis 10 Lipiddoppelschichten, die einen amorphen, zentralen Hohlraum umschließen, aufweist, wobei die Nichtphospholipidmaterialien ausgewählt sind aus der Gruppe, bestehend aus Polyoxyethylenfettsäurestern, Polyoxyethylenfettsäurethern, Polyoxyethylensorbitanestern, Polyoxyethylengycerylmono- und Diestern, Glycerylmono- und Distearat, Saccharosedistearat, Propylenglycolstearat, langkettige Acylhexosamide, langkettige Acylaminosäureamide, langkettige Acylamide, Glycerylmono- und Diester, Dimethylacylamine, C₁₂ bis C₂₀ Fettalkohole, C₁₂ bis C₂₀ Glycolmonoester, C₁₂ bis C₂₀ Fettsäuren und Mischungen dieser Nichtphospholipidmaterialien, bildet; und den Hilfsstoff mit Influenzavirusantigen, abgeleitet von formalininaktivierten Gesamtviren oder Untereinheiten davon, mischt.

13. Verfahren nach Anspruch 12, **dadurch gekennzeichnet, dass** das Antigen in den amorphen, zentralen Höhlraum eingekapselt ist.

14. Verfahren zur Herstellung eines Influenzaimpfstoffs mit Hilfsstoff, wobei man:
eine lipophile Phase, die Nichtphospholipidmaterial enthält, wobei die Nichtphospholipidmaterialien ausgewählt sind aus der Gruppe, bestehend aus Polyoxyethylenfettsäurestern, Polyoxyethylenfettsäurethern, Polyoxyethylensorbitanestern, Polyoxyethylengycerylmono- und Diestem, Glycerylmono- und Distearat, Saccharosedistearat, Propylenglycolstearat, langkettige Acylhexosamide, langkettige Acylaminosäureamide, langkettige Acylamide, Glycerylmono- und Diester, Dimethylacylamine, C₁₂ bis C₂₀ Fettalkohole, C₁₂ bis C₂₀ Glycolmonoester, C₁₂ bis C₂₀ Fettsäuren und Mischungen dieser Nichtphospholipidmaterialien, herstellt,
eine wässrige Phase, die das Influenzavirusantigen, abgeleitet aus formalininaktivierten Gesamtviren oder Untereinheiten davon, herstellt und
die lipophile Phase mit der wässrigen Phase schermischt, um die paucilamellaren Lipidvesikel mit den 2 bis 10 Lipiddoppelschichten, die einen amorphen Hohlraum umschließen, zu bilden, wobei das Antigen in den Hohlraum eingekapselt ist.

15. Verfahren nach Anspruch 12 oder Anspruch 14, **dadurch gekennzeichnet, dass** der amorphe, zentrale Hohlraum der paucilamellaren Lipidvesikel ein mit Wasser nicht mischbares, öliges Material enthält, beispielsweise ausgewählt aus der Gruppe, bestehend aus Sojabohnenöl, Squalenöl, Sesamöl, Olivenöl, Canolaöl, Maisöl, Rapsöl, Distelöl, Sonnenblumenöl, Fischölen, Petrolat, Avocadoöl, Triglycerid Öle und Fette, Duftöle, wasserunlöslichen Vitaminen und Mischungen daraus.

16. Verfahren zur Herstellung eines Impfstoffs mit Hilfsstoff, wobei der Hilfsstoff ein paucilamellares Lipidvesikel mit einem im wesentlichen amorphen, zentralen Hohlraum, der mit Wasser nicht mischbare, ölige Materialien enthält, aufweist, wobei 2 bis 10 Doppelschichten den amorphen, zentralen Hohlraum und ein Influenzavirusantigen, abgeleitet aus formalindeaktivierten Gesamtviren oder Untereinheiten davon, umgeben, wobei das paucilamellare Lipidvesikel Nichtphospholipidmaterialien aufweist, ausgewählt aus der Gruppe, bestehend aus Polyoxyethylenfettsäurestern, Polyoxyethylenfettsäurethern, Polyoxyethylensorbitanestern, Polyoxyethylengycerylmono- und Diestern, Glycerylmono- und Distearat, Saccharosedistearat, Propylenglycolstearat, langkettige Acylhexosamide, langkettige Acylaminosäureamide, langkettige Acylamide, Glycerylmono- und Diester, Dimethylacylamine, C₁₂ bis C₂₀ Fettalkohole, C₁₂ bis C₂₀ Glycolmonoester, C₁₂ bis C₂₀ Fettsäuren und Mischungen daraus, wobei man bei dem Verfahren:
ein paucilamellares Lipidvesikel mit einem wässrigen Material im amorphen, zentralen Hohlraum herstellt, wobei das paucilamellare Lipidvesikel Nichtphpspholipidmaterialien aufweist, ausgewählt aus der Gruppe, bestehend aus Polyoxyethylenfettsäurestern, Polyoxyethylenfettsäurethern, Polyoxyethylensorbitanestern, Polyoxyethylengycerylmono- und Diestern, Glycerylmono- und Distearat, Saccharosedistearat, Propylenglycolstearat, langkettige Acylhexosamide, langkettige Acylaminosäureamide, langkettige Acylamide, Glycerylmono- und Diester, Dimethylacylamine, C₁₂ bis C₂₀ Fettalkohole, C₁₂ bis C₂₀ Glycolmonoester, C₁₂ bis C₂₀ Fettsäuren und Mischungen aus diesen Nichtphospholipidmaterialien; und
unter Schermischbedingungen die hergestellten paucilamellaren Lipidvesikel mit einem nicht wassermischbaren, öligen Material, das ein Influenzavirusantigen enthält, das in den zentralen Hohlraum so eingebracht werden soll, so mischt dass das nicht wassermischbare Material und das Influenzavirusantigen in das hergestellte Vesikel eingeschlossen sind, wobei der amorphe paucilamellare Hohlraum des paucilamellaren Lipidvesikels im wesentlichren mit dem nicht wassermischbaren Material gefüllt ist.

17. Verfahren nach Anspruch 16, **dadurch gekennzeichnet, dass** das Verfahren des weiteren den Schritt aufweist, bei dem man die paucilamellaren Lipidvesikel von jedwedem des nicht wassermischbaren Materials und Antigens trennt, das nicht in die paucilamellaren Lipidvesikel eingeschlossen ist.

18. Verfahren nach Anspruch 16, **dadurch gekennzeichnet, dass** das paucilamellare Lipidvesikel 2 bis 10 Doppelschichten aufweist, die einen amorphen, zentralen Hohlraum umschließen.

19. Verfahren nach Anspruch 12, 14 oder 16, **dadurch gekennzeichnet, dass** das Antigen ausgewählt ist aus der Gruppe, bestehend aus Antigenen, die aus formalindeaktivierten Gesamtviren abgeleitet sind, Antigenen, die aus formalindeaktivierten Virusuntereinheiten abgeleitet sind und beispielsweise das Antigen ausgewählt ist aus der Gruppe, bestehend aus formalindeaktivierten, mit Detergenzien extrahierten Influenzaviren und Influenza A H3N2.

20. Verfahren nach Anspruch 12, 14 oder 16, **dadurch gekennzeichnet, dass** die paucilamellaren Lipidvesikel des weiteren zumindest ein Sterin, ausgewählt aus der Gruppe, bestehend aus Cholesterin, Cholesterinderivaten, Hydrocortison, Phytosterin und Mischungen daraus, aufweisen.

21. Verfahren nach Anspruch 16, **dadurch gekennzeichnet, dass** das mit Wasser nicht mischbare, ölige Material ausgewählt ist aus der Gruppe, bestehend aus Sojabohnenöl, Squalenöl, Sesamöl, Olivenöl, Canolaöl, Maisöl, Rapsöl, Distelöl, Sonnenblumenöl, Fischölen, Petrolat, Avocadoöl, Triglycerid Öle und Fette, Duftöle, wasserunlöslichen Vitaminen und Mischungen daraus.

## Revendications

1. Vaccin destiné à induire une réponse antigénique vis-à-vis de l'influenza, *in vivo,* chez les mammifères, ledit vaccin comprenant :
une quantité efficace sur le plan antigénique d'un antigène du virus influenza dérivé d'un virus entier inactivé au formol ou de sous-unités de celui-ci et un adjuvant, ledit adjuvant comprenant des vésicules lipidiques pauci-lamellaires contenant des matériaux nonphospholipidiques en tant que constituant principal formant la paroi, où lesdites vésicules lipidiques pauci lamellaires possèdent de 2 à 10 bicouches entourant une cavité centrale amorphe, et
où lesdits matériaux non phospholipidiques sont selectionnés dans le groupe constitué des esters d'acides gras polyoxyéthylèniques, éthers d'acides gras polyoxyéthylènéniques, esters de polyoxyéthylène sorbitane, mono et diesters de glycéryle polyoxyéthylène, mono-et distéarate de glycéryle, distéarate de saccharose, stéarate de propylène glycol, hexosamides d'acyle à longue chaîne, amides d'acides aminés acylés à longue chaîne, amides acylés à longue chaîne, de mono et diesters de glycéryle, amines de diméthylacyle, d'alcools gras de C₁₂ à C₂₀, monoesters de glycol de C₁₂ à C₂₀, acides gras de C₁₂ à C₂₀, et des mélanges desdits matériaux non phospholipidiques.

2. Vaccin selon la revendication 1 où ledit antigène et ledit adjuvant sont mélangés dans ledit vaccin, ou ledit antigène est encapsulé dans ledit adjuvant, par exemple est encapsulé dans ladite cavité centrale amorphe.

3. Vaccin selon la revendication 1 où ledit antigène est choisi au sein du groupe constitué d'antigènes dérivés de virus entiers inactivés au formol, d'antigènes dérivés de sous-unités virales inactivées au formol et, par exemple,
ledit antigène est sélectionné dans le groupe constitué par des virus influenza extraits par un détergent inactivés au formol et par le virus influenza A H3N2.

4. Vaccin selon la revendication 1 où lesdites vésicules lipidiques paucilamellaires comprennent en outre au moins un stérol sélectionné dans le groupe constitué par le cholestérol, les dérivés du cholestérol, l'hydrocortisone, le phytostérol et des mélanges de ceux-ci.

5. Vaccin selon la revendication 1 où lesdites vésicules lipidiques pauci lamellaires comprennent une cavité centrale amorphe contenant un matériau huileux non miscible dans l'eau, par exemple
sélectionné dans le groupe constitué par l'huile de soja, le squalène huileux, l'huile de sésame, l'huile d'olive, l'huile de colza canola, l'huile de maïs, l'huile de colza, l'huile de carthame, l'huile de tournesol, les huiles de poisson, le petrolatum, l'huile d'avocat, les huiles et graisses de triglycérides, les huiles aromatiques, les vitamines insolubles dans l'eau et des mélanges de celles-ci.

6. Utilisation pour la fabrication d'un vaccin destiné à l'immunisation d'un mammifère contre l'influenza, d'un antigène du virus influenza dérivé d'un virus entier inactivé au formol ou de sous-unités de celui-ci et d'un adjuvant, où ledit adjuvant comprend des vésicules paucilamellaires non phospholipidiques, lesdites vésicules paucilamellaires non phospholipidiques possédant de 2 à 10 bicouches entourant une cavité centrale amorphe et comprenant des matériaux non phospholipidiques sélectionnés dans le groupe constitué d'esters des acides gras polyoxyéthylèniques, éthers d'acides gras polyoxyéthylènéniques, esters de polyoxyéthylène sorbitane, mono et diesters de glycéryle polyoxyéthylène, mono-et distéarate de glycéryle, distéarate de saccharose, stéarate de propylène glycol, hexosamides d'acyle à longue chaîne, amides d'acides aminés acylés à longue chaîne, amides acylés à longue chaîne, mono et diesters de glycéryle, amines de diméthylacyle, alcools gras de C₁₂ à C₂₀, monoesters de glycol de C₁₂ à C₂₀, acides gras de C₁₂ à C₂₀, et des mélanges desdits matériaux non phospholipidiques, ledit vaccin étant sous une forme appropriée à une administration intramusculaire, intrapéritonéale ou orale.

7. Utilisation selon la revendication 6, où ledit antigène et ledit adjuvant sont mélangés dans ledit vaccin, ou ledit antigène est encapsulé dans ledit adjuvant.

8. Utilisation selon la revendication 6, où ledit antigène est encapsulé dans ladite cavité centrale amorphe.

9. Utilisation selon la revendication 6, où ledit antigène est sélectionné dans le groupe constitué par des antigènes dérivés d'un virus entier inactivé au formol et des antigènes dérivés de sous-unités virales inactivées au formol, et par exemple,
ledit antigène est sélectionné dans le groupe constitué par des virus influenza extraits par un détergent inactivés au formol et par le virus influenza A H3N2.

10. Utilisation selon la revendication 6, où lesdites vésicules lipidiques pauci lamellaires comprennent en outre au moins un stérol sélectionné dans le groupe constitué par le cholestérol, les dérivés du cholestérol, l'hydrocortisone, le phytostérol, et des mélanges de ceux-ci.

11. Utilisation selon la revendication 6, où lesdites vésicules lipidiques, pauci lamellaires comprennent une cavité centrale amorphe contenant un matériau huileux non miscible dans l'eau, par exemple sélectionné dans le groupe constitué par l'huile de soja, le squalène huileux, l'huile de sésame, l'huile d'olive, l'huile de colza canola, l'huile de maïs, l'huile de colza, l'huile de carthame, l'huile de tournesol, les huiles de poisson, le petrolatum, l'huile d'avocat, les huiles et graisses de triglycérides, les huiles aromatiques, les vitamines insolubles dans l'eau et des mélanges de celles-ci.

12. Procédé de préparation d'un vaccin contre l'influenza adjuvé, ledit procédé comprenant les étapes consistant à :
préparer un adjuvant comprenant des vésicules paucilamellaires non phospholipidiques possédant de 2 à 10 bicouches entourant une cavité centrale amorphe, lesdites vésicules paucilamellaires non phospholipidiques comprenant des matériaux non phospholipidiques sélectionnés dans le groupe constitué des esters d'acides gras polyoxyéthylèniques, éthers d'acides gras polyoxyéthylènéniques, esters de polyoxyéthylène sorbitane, mono et diesters de glycéryle polyoxyéthylène, mono-et distéarate de glycéryle, distéarate de saccharose, stéarate de propylène glycol, hexosamides d'acyle à longue chaîne, amides d'acides aminés acylés à longue chaîne, amides acylés à longue chaîne, mono et diesters de glycéryle, amines de diméthylacyle, alcools gras de C₁₂ à C₂₀, monoesters de glycol de C₁₂ à C₂₀, acides gras de C₁₂ à C₂₀, et des mélanges desdits matériaux non phospholipidiques ; et à mélanger ledit adjuvant avec un antigène de virus influenza dérivé d'un virus entier inactivé par le formol ou de sous-unités de celui-ci.

13. Procédé selon la revendication 12 dans lequel ledit antigène est encapsulé dans ladite cavité centrale amorphe dudit adjuvant.

14. Procédé de préparation d'un vaccin contre l'influenza adjuvé, ledit procédé comprenant les étapes consistant à :
préparer une phase lipophile contenant un matériau non phospholipidique où ledit matériau non phospholipidique est sélectionné dans le groupe constitué des esters d'acides gras polyoxyéthylèniques, éthers d'acides gras polyoxyéthylènéniques, esters de polyoxyéthylène sorbitane, mono et diesters de glycéryle polyoxyéthylène, mono-et distéarate de glycéryle, distéarate de saccharose, stéarate de propylène glycol, hexosamides d'acyle à longue chaîne, amides d'acides aminés acylés à longue chaîne, amides acylés à longue chaîne, mono et diesters de glycéryle, d'amines de diméthylacyle, alcools gras de C₁₂ à C₂₀, monoesters de glycol de C₁₂ à C₂₀, acides gras de C₁₂ à C₂₀, et des mélanges desdits matériaux nonphospholipidiques ;
préparer une phase aqueuse contenant un antigène de virus influenza dérivé d'un virus entier inactivé au formol ou de sous unités de celui-ci ; et
mélanger par cisaillement ladite phase lipophile avec ladite phase aqueuse pour former des vésicules lipidiques paucilamellaires possédant 2 à 10 bicouches lipidiques entourant une cavité centrale amorphe, où ledit antigène est encapsulé dans ladite cavité.

15. Procédé selon la revendication 12 ou 14, où ladite cavité centrale amorphe desdites vésicules lipidiques paucilamellaires contient un matériau huileux non miscible dans l'eau, par exemple sélectionné dans le groupe constitué par l'huile de soja, le squalène huileux, l'huile de sésame, l'huile d'olive, l'huile de colza canola, l'huile de maïs, l'huile de colza, l'huile de carthame, l'huile de tournesol, les huiles de poisson, le petrolatum, l'huile d'avocat, les huiles et graisses de triglycérides, les huiles aromatiques, les vitamines insolubles dans l'eau et des mélanges de celles-ci.

16. Procédé de préparation d'un vaccin adjuvé où ledit adjuvant comprend une vésicule lipidique paucilamellaire possédant une cavité centrale essentiellement amorphe contenant un matériau huileux non miscible dans l'eau, de 2 à 10 bicouches entourant ladite cavité centrale amorphe et un antigène de virus influenza dérivé d'un virus entier inactivé au formol ou de sous-unités de celui-ci, où ladite vésicule lipidique paucilamellaire comprend des matériaux non phospholipidiques sélectionnés dans le groupe constitué d'esters d'acides gras polyoxyéthylèniques, d'éthers d'acides gras polyoxyéthylènéniques, d'esters de polyoxyéthylène sorbitane, de mono et diesters de glycéryle polyoxyéthylène, de mono- et distéarate de glycéryle, de distéarate de saccharose, de stéarate de propylène glycol, d'hexosamides d'acyle à longue chaîne, d'amides d'acides aminés acylés à longue chaîne, d'amides acylées à longue chaîne, de mono et diesters de glycéryle, d'amines de diméthylacyle, d'alcools gras de C₁₂ à C₂₀, de monoesters de glycol de C₁₂ à C₂₀, d'acides gras de C₁₂ à C₂₀ et des mélanges de ceux-ci, ledit procédé comprenant les étapes consistant à :
préformer une vésicule lipidique pauci lamellaire possédant un matériau aqueux dans la cavité centrale amorphe, ladite vésicule lipidique pauci-lamellaire comprenant des matériaux non phospholipidiques sélectionnés dans le groupe constitué des esters d'acides gras polyoxyéthylèniques, éthers d'acides, gras polyoxyéthylènéniques, esters de polyoxyéthylène sorbitane, mono et diesters de glycéryle polyoxyéthylène, mono-et distéarate de glycéryle, distéarate de saccharose, stéarate de propylène glycol, hexosamides d'acyle à longue chaîne, amides d'acides aminés acylés à longue chaîne, amides acylés à longue chaîne, mono et diesters de glycéryle, amines de diméthylacyle, alcools gras de C₁₂ à C₂₀, monoesters de glycol de C₁₂ à C₂₀, acides gras de C₁₂ à C₂₀ ; et des mélanges desdits matériaux nonphospholipidiques ; et à
mélanger dans des conditions de mélange sous cisaillement ladite vésicule lipidique pauci lamellaire préparée au préalable avec un matériau huileux non miscible dans l'eau contenant un antigène de virus influenza à incorporer dans ladite cavité centrale de telle sorte que ledit matériau non miscible dans l'eau et ledit antigène de virus influenza se trouvent incorporés dans ladite vésicule préparée au préalable, de manière à ce que ladite cavité centrale amorphe de ladite vésicule lipidique pauci lamellaire soit essentiellement remplie avec ledit matériau non miscible dans l'eau.

17. Procédé selon la revendication 16, comprenant en outre une étape de séparation de ladite vésicule lipidique paucilamellaire de tout dit matériau non miscible dans l'eau et antigène non incorporé dans ladite vésicule lipidique paucilamellaire.

18. Procédé selon la revendication 16, où lesdites vésicules lipidiques paucilamellaires possèdent de 2 à 10 bicouches entourant une cavité centrale amorphe.

19. Procédé selon la revendication 12, 14 ou 16 où ledit antigène est sélectionné dans le groupe constitué d'antigènes dérivés d'un virus entier inactivé au formol, d'antigènes dérivés de sous-unités virales inactivées au formol et ledit antigène est par exemple sélectionné dans le groupe constitué par des virus influenza extraits par un détergent inactivés au formol et le virus influenza de souche A H3N2.

20. Procédé selon la revendication 12, 14 ou 16, où ladite vésicule lipidique paucilamellaire ou ladite phase lipophile comprend en outre au moins un stérol sélectionné dans le groupe constitué par le cholestérol, les dérivés du cholestérol, l'hydrocortisone, le phytostérol ou des mélanges de ceux-ci.

21. Procédé de la revendication 16, dans lequel ledit matériau huileux non miscible dans l'eau est sélectionné dans le groupe constitué par l'huile de soja, le squalème huileux, l'huile de sésame, l'huile d'olive, l'huile de colza canola, l'huile de maïs, l'huile de colza, l'huile de carthame, l'huile de tournesol, les huiles de poisson, le petrolatum, l'huile d'avocat, les huiles et graisses de triglycérides, les huiles aromatiques, les vitamines insolubles dans l'eau et des mélanges de celles-ci.
